(19) Europäisches Patentamt
European Patent Office
Office européen des brevets

(11) **EP 4 313 349 B1**

(12) **FASCICULE DE BREVET EUROPEEN**

(45) Date de publication et mention
de la délivrance du brevet:
**06.05.2026 Bulletin 2026/19**

(21) Numéro de dépôt: **22714845.9**

(22) Date de dépôt: **15.03.2022**

(51) Classification Internationale des Brevets (IPC):
***B01D 15/18*** *(2006.01)* ***C07C 7/12*** *(2006.01)*
***C07C 15/08*** *(2006.01)*

(52) Classification Coopérative des Brevets (CPC):
**B01D 15/1842**

(86) Numéro de dépôt international:
**PCT/EP2022/056646**

(87) Numéro de publication internationale:
**WO 2022/200119 (29.09.2022 Gazette 2022/39)**

(54) **DISPOSITIF ET PROCÉDÉ POUR LA SÉPARATION EN LIT MOBILE SIMULÉ**

VORRICHTUNG UND VERFAHREN ZUR SIMULIERTEN WANDERBETTTRENNUNG

DEVICE AND METHOD FOR SIMULATED MOVING BED SEPARATION

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **25.03.2021 FR 2103022**

(43) Date de publication de la demande:
**07.02.2024 Bulletin 2024/06**

(73) Titulaire: **IFP Energies nouvelles**
**92500 Rueil-Malmaison (FR)**

(72) Inventeurs:
• **VONNER, Alexandre**
**92852 RUEIL-MALMAISON CEDEX (FR)**
• **LEINEKUGEL-LE-COCQ, Damien**
**92852 RUEIL-MALMAISON CEDEX (FR)**
• **BLANCKE, Guillaume**
**92852 RUEIL-MALMAISON CEDEX (FR)**
• **AUGIER, Frédéric**
**92852 RUEIL-MALMAISON CEDEX (FR)**
• **ROYON-LEBEAUD, Aude**
**92852 RUEIL-MALMAISON CEDEX (FR)**
• **FOURATI, Manel**
**92852 RUEIL-MALMAISON CEDEX (FR)**
• **AHMADI-MOTLAGH, Amir Hossein**
**92852 RUEIL-MALMAISON CEDEX (FR)**

(74) Mandataire: **IFP Energies nouvelles**
**Département Propriété Industrielle**
**Rond Point de l'échangeur de Solaize**
**BP3**
**69360 Solaize (FR)**

(56) Documents cités:
EP-A1- 2 319 599 WO-A1-2008/048395
FR-A1- 3 010 911 GB-A- 1 418 503

# Description

## Domaine technique

La présente invention concerne un dispositif et un procédé pour la séparation en lit mobile simulé, notamment pour la séparation du paraxylène d'un mélange de composés aromatiques comprenant 8 atomes de carbones (orthoxylène, métaxylène et éthylbenzène).

## Technique antérieure

Les technologies actuelles de séparation en lit mobile simulé (parfois en abrégé dans la suite du texte LMS ou SMB pour « Simulated Moving Bed » selon la terminologie anglo-saxonne) utilisent des unités qui comportent un certain nombre de points communs :

- un ou deux adsorbeurs (ou colonne de séparation) comprenant chacun une pluralité de chambres d'adsorption disposées entre un canal de distribution et un canal de collecte, lesdites chambres d'adsorption comprenant un lit d'adsorbant au sein duquel s'écoule un fluide,

- des systèmes d'injection, notamment de la charge et de désorbant, et de soutirage, notamment des effluents produits appelés extrait et raffinat,

- des systèmes de collecte et redistribution, dits zones inter-lits, pour passer d'un lit au lit suivant.

Un problème de la technologie de séparation en LMS actuelle est que les adsorbeurs comportent un grand nombre de lits (typiquement 12 lits ou plus) avec un ratio hauteur H sur diamètre D faible (ratio typiquement bien inférieur à 1), afin que l'adsorbeur ne soit pas trop haut et ainsi difficile à ériger, utiliser et entretenir.

Un autre problème de la technologie de séparation en LMS actuelle est que l'écoulement des fluides dans les lits des adsorbeurs est de haut en bas, les fluides du fond de l'adsorbeur étant remontés vers la tête de l'adsorbeur ou de l'adsorbeur suivant, ce qui nécessite un investissement en énergie et en équipement (vannes, pompes...) conséquent pour pousser des milliers de $m^3/h$ de fluide sur plus d'une dizaine de mètres de haut.

Un autre problème de la technologie de séparation en LMS actuelle est que les adsorbeurs comportent des grands volumes des zones dites inter-lits ce qui bride la capacité de séparation et augmente la nécessité d'utiliser de lourds équipements. Un autre problème de la technologie de séparation en LMS actuelle est que tous les lits (e.g. 12 lits) d'un même adsorbeur nécessitent d'être arrêtés afin de maintenir ou réparer une section prédéterminée de l'adsorbeur.

## Résumé de l'invention

Dans le contexte précédemment décrit, un premier objet de la présente description est de surmonter les problèmes de l'art antérieur et de fournir un dispositif et un procédé de séparation en LMS étant plus simple à ériger, utiliser et entretenir.

Un deuxième objet de la présente description est fournir un dispositif et un procédé de séparation en LMS permettant une économie en énergie et en équipement.

Un troisième objet de la présente description est fournir un dispositif et un procédé de séparation en LMS permettant d'isoler seulement une partie des lits (e.g. un lit ou une paire de lits), sans arrêt du reste du dispositif, qui peut fonctionner dans un mode repli, avec des performances suffisantes.

Selon un premier aspect de l'invention, les objets précités, ainsi que d'autres avantages, sont obtenus par un dispositif de séparation en lit mobile simulé comprenant une pluralité d'adsorbeurs disposés en série agencés alternativement en courant ascendant et descendant, chaque adsorbeur étant divisé en n chambres d'adsorption comprenant chacune un lit d'adsorbant, les n lits d'adsorbant étant séparés par n plateaux pour injecter une charge et un désorbant et soutirer un extrait et un raffinat, le dispositif comprenant au moins une ligne de court-circuit adaptée pour court-circuiter au moins un adsorbeur.

Selon un ou plusieurs modes de réalisation, le nombre d'adsorbeurs m est compris entre 3 et 15, préférablement compris entre 4 et 12, très préférablement compris entre 5 et 10.

Selon un ou plusieurs modes de réalisation, le nombre d'adsorbeurs m est un nombre pair.

Selon un ou plusieurs modes de réalisation, le nombre n de lits d'adsorbant par adsorbeur est compris entre 1 et 4, préférablement compris entre 1 et 3, très préférablement compris entre 1 et 2.

Selon un ou plusieurs modes de réalisation, le nombre total t de lits d'adsorbant est compris entre 6 et 24, préférablement compris entre 8 et 19, très préférablement compris entre 12 et 15.

Selon un ou plusieurs modes de réalisation, les lits d'adsorbants présentent un ratio H/D hauteur H sur diamètre D égal à ou supérieur à 1, préférablement égal à ou supérieur à 1,5, très préférablement égal à ou supérieur à 2.

Selon un ou plusieurs modes de réalisation, les lits d'adsorbants présentent un ratio H/D hauteur H sur diamètre D compris entre 1 et 12, préférablement compris entre 1,5 et 10, très préférablement compris entre 2 et 8.

Selon un ou plusieurs modes de réalisation, la ligne de court-circuit est adaptée pour court-circuiter deux adsorbeurs adjacents.

Selon un ou plusieurs modes de réalisation, la ligne de court-circuit connecte un adsorbeur à courant descendant à un adsorbeur à courant ascendant.

Selon un ou plusieurs modes de réalisation, les adsorbeurs sont agencés en disposition verticale pour une distribution sensiblement verticale du fluide circulant dans les adsorbeurs.

Selon un ou plusieurs modes de réalisation, les adsorbeurs sont agencés en disposition horizontale pour une distribution sensiblement horizontale du fluide circulant dans les adsorbeurs. Avantageusement, la circulation (ascendante/descendante) dans les adsorbeurs peut se faire dans un plan horizontal et suivant deux directions opposées.

Selon un deuxième aspect de l'invention, les objets précités, ainsi que d'autres avantages, sont obtenus par un procédé de séparation en lit mobile simulé utilisant le dispositif de séparation en lit mobile simulé selon le premier aspect, le procédé comprenant les étapes suivantes :

- on alimente les adsorbeurs avec au moins une charge et un désorbant, et on soutire au moins un extrait et au moins un raffinat desdits adsorbeurs, les lits d'adsorbant étant interconnectés en boucle fermée, les points d'alimentation et de soutirage dans les adsorbeurs étant décalés au cours du temps d'une valeur correspondant à un lit d'adsorbant avec une période de permutation et déterminant une pluralité de zones de fonctionnement du dispositif de séparation en lit mobile simulé, et notamment les zones principales suivantes :

- la zone I de désorption d'un produit (d'intérêt) à séparer (e.g. paraxylène) est comprise entre l'injection du désorbant D et le soutirage de l'extrait E ;

- la zone II de désorption des isomères du produit à séparer est comprise entre le soutirage de l'extrait E et l'injection de la charge F ;

- la zone III d'adsorption du produit à séparer est comprise entre l'injection de la charge F et le soutirage du raffinat R ; et

- la zone IV est comprise entre le soutirage de raffinat R et l'injection de désorbant D.

Selon un ou plusieurs modes de réalisation, les lits d'adsorbant sont répartis dans les zones I à IV selon des configurations dites de type a / b / c / d suivantes :

- a est le nombre de lits en zone I ;
- b est le nombre de lits en zone II ;
- c est le nombre de lits en zone III ; et
- d est le nombre de lits en zone IV, et
-

$$a = (t * 0,2) * (1 \pm 0,2) ;$$

-

$$b = (t * 0,4) * (1 \pm 0,2) ;$$

-

$$c = (t * 0,27) * (1 \pm 0,2) ;$$

et

-

$$d = (t * 0,13) * (1 \pm 0,2),$$

et dans lequel t est un nombre entier naturel compris entre 6 et 24, préférablement entre 8 et 15.

Selon un ou plusieurs modes de réalisation, les lits d'adsorbant sont répartis dans les zones I à IV selon des configurations dites de type a / b / c / d suivantes :

- a est le nombre de lits en zone I ;
- b est le nombre de lits en zone II ;
- c est le nombre de lits en zone III ; et
- d est le nombre de lits en zone IV, et
-

$$a = (t * 0,17) * (1 \pm 0,2) ;$$

-

$$b = (t * 0,42) * (1 \pm 0,2) ;$$

-

$$c = (t * 0,25) * (1 \pm 0,2) ;$$

et

-

$$d = (t * 0,17) * (1 \pm 0,2), et$$

dans lequel t est un nombre entier naturel compris entre 6 et 24, préférablement entre 8 et 15.

Selon un ou plusieurs modes de réalisation, le procédé comprend au moins une des conditions opératoires suivantes :

- le désorbant est choisi parmi le groupe constitué par un ou plusieurs isomères de diéthylbenzène et le toluène ;

- l'adsorbant utilisé comprend/consiste en une Faujasite choisi parmi le groupe consistant en BaX, BaKX, et BaLSX ;

- la charge est choisie parmi le groupe constitué par un mélange de composés essentiellement aromatiques en C8 ;

- la température dans les lits d'adsorbant est comprise entre 140°C et 189°C ;

- la pression est contrôlée pour rester en phase liquide en tout point du dispositif ;

- la période de permutation ST est comprise entre 20 secondes et 90 secondes ; et

- le débit de circulation moyen entre les lits est compris entre 1000 et 5000 m³/h.

D'autres caractéristiques et avantages de l'invention selon les aspects précités, apparaîtront à la lecture de la description ci-après et d'exemples non limitatifs de réalisations, en se référant aux figures annexées et décrites ci-après.

**Liste des figures**

La figure 1 montre schématiquement un dispositif de séparation en LMS de référence dans lequel les adsorbeurs sont à courant descendant.

La figure 2 montre schématiquement un dispositif de séparation en LMS selon un ou plusieurs modes de réalisation de l'invention dans lequel les adsorbeurs sont alternativement à courant descendant et courant ascendant.

**Description des modes de réalisation**

L'invention porte sur un dispositif et un procédé pour la séparation en lit mobile simulé, notamment pour la séparation du paraxylène.
Des modes de réalisation du dispositif et du procédé selon les aspects précités vont maintenant être décrits en détail. Dans la description détaillée suivante, de nombreux détails spécifiques sont exposés afin de fournir une compréhension plus approfondie du procédé. Cependant, il apparaîtra à l'homme du métier que le procédé peut être mis en œuvre sans ces détails spécifiques. Dans d'autres cas, des caractéristiques bien connues n'ont pas été décrites en détail pour éviter de compliquer inutilement la description.
Dans la présente demande, le terme « comprendre » est synonyme de (signifie la même chose que) « inclure » et « contenir », et est inclusif ou ouvert et n'exclut pas d'autres éléments non récités. Il est entendu que le terme « comprendre » inclut le terme exclusif et fermé « consister ». En outre, dans la présente description, les termes « essentiellement » ou « sensiblement » correspondent à une approximation de ± 5%, préférablement de ±1%, très préférablement de ± 0,5%. Par exemple, un effluent comprenant essentiellement ou étant constitué des composés A correspond à un effluent comprenant au moins 95% en poids de composés A.
La présente invention peut se définir comme un dispositif ou une unité comprenant une pluralité d'adsorbeurs (ou colonnes de séparation) disposés en série pour la séparation en lit mobile simulé de composés (e.g. xylènes), chaque adsorbeur étant divisé en n chambres d'adsorption comprenant chacune un lit d'adsorbant, les n lits d'adsorbant étant séparés par n plateaux ou zones inter-lits (*i.e.,* n zones de distribution et n zones de collecte), dans lequel les adsorbeurs sont agencés alternativement en courant ascendant et descendant. Les zones de distribution et de collecte comprennent des systèmes de collecte et redistribution pour transmettre le fluide traversant l'adsorbeur d'un lit au lit suivant.
Dans la présente demande, on considère que la première zone de distribution du premier lit d'adsorbant et la dernière zone de collecte du dernier lit d'adsorbant forment ensemble une même zone inter-lits.

*Le dispositif*

Un dispositif de séparation en lit mobile simulé selon l'invention est, par exemple, une colonne chromatographique ou colonne d'adsorption, opérant en lit mobile simulé. La séparation en lit mobile simulé est une technique bien connue. En règle générale, la colonne fonctionnant en lit mobile simulé comporte au moins trois zones, généralement quatre, et éventuellement cinq, chacune de ces zones comportant un certain nombre de lits d'adsorbant successifs (e.g. lits fixes), et chaque zone étant définie par sa position comprise entre un point d'alimentation et un point de soutirage. Typiquement, une colonne en lit mobile simulé est alimentée par au moins une charge F (mélange de xylènes) à fractionner et un désorbant D (parfois appelé éluant), et l'on soutire de ladite colonne au moins un raffinat R (mélange de xylènes appauvri en paraxylène) et un extrait E (désorbant et paraxylène). Les points d'alimentation et de soutirage sont modifiés au cours du temps, typiquement décalés dans le même sens d'une valeur correspondant à un lit d'adsorbant.
Avantageusement, l'agencement alternatif en courant ascendant/descendant des adsorbeurs du dispositif et du procédé selon la présente invention, permet d'améliorer la construction de l'unité, de simplifier et limiter les équipements nécessaires, d'améliorer la séparation des fluides, et de réduire les coûts énergétiques de fonctionnement par la diminution du volume des zones dites inter-lits. De plus, la présente invention permet la maintenabilité de l'unité avec la possibilité d'isoler totalement seulement une paire de lits, sans arrêt du reste de l'unité, qui peut fonctionner dans un mode repli, avec des performances acceptables, bien que parfois réduites.

L'état de la technique décrit de façon approfondie différents dispositifs permettant d'effectuer la séparation de charges en lit mobile simulé. On peut citer notamment les brevets US 2,985,589, US 3,214,247, US 3,268,605, US 3,592,612, US 4,614,204, US 4,378,292, US 5,200,075, US 5,316,821 ainsi que EP 2319599 A1.

Selon un ou plusieurs modes de réalisation, les zones de distribution et de collecte comprennent des systèmes d'injection, notamment d'une charge (e.g. mélange de xylènes) et d'un désorbant (e.g. toluène or paradiéthylbenzène), et de soutirage, notamment des effluents produits appelés extrait (e.g. mélange de paraxylène et de désorbant) et raffinat (e.g. mélange d'orthoxylène, de métaxylène, de désorbant et optionnellement d'éthylbenzène).

Les moyens/dispositifs commandés d'alimentation et de soutirage de fluides d'un dispositif de séparation en lit mobile simulé sont par exemple l'un des deux grands types suivants de technologie :

- soit, pour chaque plateau, une pluralité de vannes commandées tout ou rien (optionnellement avec des éléments de régulation des débits) pour l'alimentation ou le soutirage des fluides, ces vannes étant typiquement situées au voisinage immédiat du plateau correspondant. Chaque plateau comprend typiquement au moins quatre vannes à deux voies, commandées en tout ou rien, pour effectuer respectivement les alimentations de la charge et du désorbant et les soutirages de l'extrait et du raffinat ;

- soit une vanne rotative multivoies pour l'alimentation ou le soutirage des fluides sur l'ensemble des plateaux.

La présente invention se situe notamment dans le cadre des colonnes fonctionnant en lit mobile simulé utilisant une pluralité de vannes pour assurer l'alimentation et le soutirage des différents fluides.

Préférablement, le nombre m d'adsorbeurs est compris entre 3 et 15, préférablement compris entre 4 et 12, très préférablement compris entre 5 et 10. Préférablement, le nombre m d'adsorbeurs est un nombre pair.

Préférablement, le nombre n de lits d'adsorbant par adsorbeur est compris entre 1 et 4, et préférablement compris entre 1 et 3, très préférablement entre 1 et 2.

Préférablement, le nombre total t de lits d'adsorbant est compris entre 6 et 24, préférablement compris entre 8 et 19, très préférablement entre 12 et 15.

Selon un ou plusieurs modes de réalisation de la présente invention, les lits d'adsorbants présentent un ratio H/D hauteur H sur diamètre D d'au moins 1 (préférablement supérieur à 1), préférablement d'au moins 1,5, très préférablement d'au moins 2, tel que d'au moins 3 ou 4. Selon un ou plusieurs modes de réalisation de la présente invention, les lits d'adsorbants présentent un ratio H/D hauteur H sur diamètre D compris entre 1 et 12, préférablement compris entre 1,5 et 10, très préférablement compris entre 2 et 8.

Un avantage supplémentaire du dispositif et du procédé selon l'invention est la possibilité d'utiliser des lits d'adsorbants ayant un ratio H/D hauteur H sur diamètre D plus grand que l'art antérieur et donne accès à l'utilisation de lits radiaux qui permettent une séparation améliorée.

Le dispositif selon l'invention comprend en outre une ligne de court-circuit adaptée pour court-circuiter au moins un adsorbeur, par exemple deux adsorbeurs du dispositif. Avantageusement, il est possible d'isoler seulement une partie des lits d'adsorbants pour maintenance, par exemple en isolant un ou deux adsorbeurs successifs, et de continuer le procédé de séparation avec des performances acceptables, et améliorées en comparaison avec par exemple l'arrêt d'un adsorbeur à 12 lits.

Selon un ou plusieurs modes de réalisation, la ligne de court-circuit connecte un adsorbeur i amont (i étant compris entre 1 et m) à un adsorbeur i+2 aval ou un adsorbeur i+3 aval. Selon un ou plusieurs modes de réalisation, la ligne de court-circuit est adaptée pour court-circuiter, par exemple au moyen d'une vanne de dérivation, l'adsorbeur i+1 et/ou l'adsorbeur i+2 en envoyant les flux sortant de l'adsorbeur i vers l'adsorbeur i+2 ou l'adsorbeur i+3. Selon un ou plusieurs modes de réalisation, la ligne de court-circuit connecte un adsorbeur i à courant descendant à un adsorbeur i+3 à courant ascendant.

Selon un ou plusieurs modes de réalisation, les adsorbeurs sont disposés dans une disposition verticale. L'invention s'applique aussi dans le cas d'une disposition horizontale des adsorbeurs. Alors la circulation se fait dans le plan horizontal et suivant deux directions opposées. Le concept reste identique à l'écoulement ascendant/descendant et le gain associé est le même.

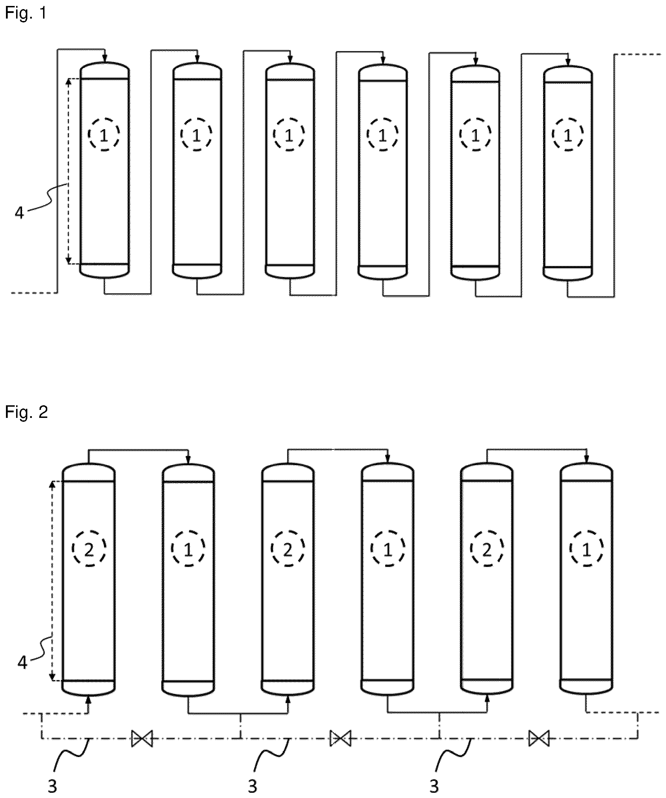

En référence à la figure 1, un dispositif pour la séparation en LMS de référence comprend une pluralité d'adsorbeurs à courant descendant 1, chaque adsorbeur comprenant une virole disposée entre un dôme supérieur et un dôme inférieur. L'agencement à courant descendant du dispositif de référence correspond à la circulation des fluides globalement descendante dans tous les adsorbeurs au moyen d'une ligne connectant le fond d'un adsorbeur i à la tête de l'adsorbeur i+1 suivant. Généralement, les dispositifs de référence sont des unités à 24 lits comprenant deux adsorbeurs à courant descendant contenant chacun 12 lits.

En référence à la figure 2, un dispositif de séparation en LMS selon un ou plusieurs modes de réalisation de l'invention comprend une pluralité d'adsorbeurs agencés alternativement en courant ascendant et descendant, chaque adsorbeur comprenant une virole disposée entre un dôme supérieur et un dôme inférieur.

L'agencement alternatif des adsorbeurs à courant descendant 1 et des adsorbeurs à courant ascendant 2 du dispositif selon l'invention correspond à une circulation des fluides globalement ascendante dans des adsorbeurs i, i+2, i+4, etc... et une circulation des fluides globalement descendante dans les adsorbeurs i+1, i+3, i+5, etc...., la tête d'un adsorbeur i étant connecté à la tête de l'adsorbeur i+1 suivant et le fond de l'adsorbeur i+1 étant connecté à la tête de l'adsorbeur i+2 suivant. Le dispositif de séparation en LMS selon un ou plusieurs modes de réalisation de l'invention comprend en outre une pluralité de lignes de court-circuit 3, chaque ligne de court-circuit 3 connectant (dans le sens de l'écoulement) chacune un adsorbeur à courant descendant 1 amont à un adsorbeur à courant ascendant 2 aval, court-circuitant ainsi 2 adsorbeurs.

*Le procédé*

Dans la suite du texte, on parle d'étape pour désigner une opération ou un groupe d'opérations similaires effectuées sur un flux donné en un certain point du procédé. On décrit le procédé dans ses différentes étapes prises dans l'ordre d'écoulement des flux ou des produits.

Le procédé de séparation en LMS comprend les étapes suivantes : on alimente les adsorbeurs avec au moins une charge F et un désorbant D, et on soutire au moins un extrait E et au moins un raffinat R desdits adsorbeurs, lesdits adsorbeurs comprenant un ou plusieurs lits d'un solide adsorbant interconnectés en boucle fermée (i.e., le dernier lit du dernier adsorbeur étant adapté pour envoyer le flux circulant dans le premier lit du premier adsorbeur), les points d'alimentation et de soutirage dans les adsorbeurs étant décalés au cours du temps d'une valeur correspondant à un lit d'adsorbant avec une période de permutation (notée ST) et déterminant une pluralité de zones de fonctionnement du dispositif LMS, et notamment les zones principales suivantes :

Par définition, on désigne chacune des zones de fonctionnement par un numéro :

- la zone I de désorption du paraxylène est comprise entre l'injection du désorbant D et le soutirage de l'extrait E ;
- la zone II de désorption des isomères est comprise entre le soutirage de l'extrait E et l'injection de la charge F ;
- la zone III d'adsorption du paraxylène est comprise entre l'injection de la charge F et le soutirage du raffinat R ; et
- la zone IV est comprise entre le soutirage de raffinat R et l'injection de désorbant D.

Selon un ou plusieurs modes de réalisation, les lits d'adsorbant sont répartis dans les zones I à IV selon des configurations dites de type a / b / c / d, c'est-à-dire que la répartition des lits est la suivante :

- a est le nombre de lits en zone I ;

- b est le nombre de lits en zone II ;
- c est le nombre de lits en zone III ; et
- d est le nombre de lits en zone IV.

Selon un ou plusieurs modes de réalisation :

-

$$a = (t * 0,2) * (1 \pm 0,2) ;$$

-

$$b = (t * 0,4) * (1 \pm 0,2) ;$$

-

$$c = (t * 0,27) * (1 \pm 0,2) ;$$

et

-

$$d = (t * 0,13) * (1 \pm 0,2),$$

et

dans lequel t est un nombre entier naturel compris entre 6 et 24, préférablement entre 8 et 15 (e.g. 12).

Selon un ou plusieurs modes de réalisation :

-

$$a = (t * 0,17) * (1 \pm 0,2) ;$$

-

$$b = (t * 0,42) * (1 \pm 0,2) ;$$

-

$$c = (t * 0,25) * (1 \pm 0,2) ;$$

et

-

$$d = (t * 0,17) * (1 \pm 0,2),$$

et dans lequel t est un nombre entier naturel compris entre 6 et 24, préférablement entre 8 et 15 (e.g. 12).

En opération normale (par exemple, sans court-circuiter un adsorbeur), l'alimentation et le soutirage sont effectués avec une proportion prédéterminée du nombre de lits d'adsorbant en opération dans les zones I à IV. Par exemple, la proportion de lits d'adsorbant en zone I en opération normale est égale à a/t, t étant le nombre total de lits d'adsorbants. Selon un ou plusieurs modes de réalisation, lorsqu'on court-circuite un nombre x d'adsorbeurs, les dispositifs commandés d'alimentation et de soutirage (e.g. système à multiples vannes tout-ou-rien, vanne rotative multivoies) sont adaptés pour modifier la position des points d'alimentation et des point de soutirage de sorte que la proportion prédéterminée du nombre moyen de lits d'adsorbant en opération dans les zones I à IV soit conservée selon une approximation de ± 20%, préférablement de ±10%, très préférablement de ± 5%. Par exemple, la proportion de lits d'adsorbant en zone I en opération de court-circuitage est égale à a/(t-x).

Dans la présente description, une zone comprenant « un nombre moyen » de lits d'adsorbant correspond à une zone pouvant comprendre ponctuellement un premier nombre X (entier naturel) de lits d'adsorbant, et ponctuellement un deuxième nombre X-1 ou X+1 (entier naturel) de lits d'adsorbant. Par exemple, une zone I comprenant 2,5 lits, comprend ponctuellement 2 lits d'adsorbant et comprend ponctuellement 3 lits d'adsorbant.

Selon un ou plusieurs modes de réalisation, pendant une première partie de la période de permutation ST (e.g. lorsque des permutations des points injections et soutirages sont déphasées), au moins une zone comprend un premier nombre X de lits d'adsorbant ; et pendant une deuxième partie de la période de permutation ST, l'au moins une zone comprend un deuxième nombre X+1 ou X-1 de lits d'adsorbant.

Selon un ou plusieurs modes de réalisation, pendant un premier temps de cycle, ou une première partie d'un temps de cycle, au moins une zone comprend un premier nombre X de lits d'adsorbant ; et pendant un deuxième temps de cycle, ou une deuxième partie du temps de cycle, l'au moins une zone comprend un deuxième nombre X+1 ou X-1 de lits d'adsorbant. Dans la présente description, le temps de cycle correspond au temps pendant lequel les points injection et de soutirage de l'unité évoluent jusqu'à retrouver leur position initiale.

Selon un ou plusieurs modes de réalisation, lorsqu'un nombre x d'adsorbeurs est court circuité, l'alimentation et le soutirage sont effectués en opération de court-circuitage selon la configuration a / b / c / d suivante:

-

$$a = ((t-x) * 0,2) * (1 \pm 0,2) ;$$

-

$$b = ((t-x) * 0,4) * (1 \pm 0,2) ;$$

-

$$c = ((t-x) * 0,27) * (1 \pm 0,2) ;$$

et

-

$$d = ((t-x) * 0,13) * (1 \pm 0,2),$$

et

dans lequel t est un nombre entier naturel compris entre 6 et 24, préférablement entre 8 et 15 (e.g. 12).

Selon un ou plusieurs modes de réalisation, lorsqu'un nombre x d'adsorbeurs est court circuité, l'alimentation et le soutirage sont effectués en opération de court-circuitage selon la configuration a / b / c / d suivante:

-

$$a = ((t-x) * 0,17) * (1 \pm 0,2) ;$$

-

$$b = ((t-x) * 0,42) * (1 \pm 0,2) ;$$

-

$$c = ((t-x) * 0,25) * (1 \pm 0,2) ;$$

et

-

$$d = ((t-x) * 0,17) * (1 \pm 0,2),$$

et dans lequel t est un nombre entier naturel compris entre 6 et 24, préférablement entre 8 et 15 (e.g. 12).

Préférablement, le nombre x d'adsorbeurs court-circuités est égale à 1 ou 2, très préférablement, x est égal à 2.

Considérons par exemple un dispositif de séparation en lit mobile simulé de référence, tel qu'une tour comprenant 12 lits empilés dans une même virole pour la séparation de xylènes. En cas de disfonctionnement d'un lit (fuite, casse, ...) sur une tour de 12 lits, il convient d'arrêter l'ensemble de la tour, de la décharger et la recharger avant redémarrage. Cela implique de longues périodes pendant lesquelles le dispositif est arrêté et où il n'y pas de production.

Considérons maintenant un dispositif de l'invention, comprenant par exemple 12 adsorbeurs pour la séparation de xylènes dans lequel un lit est défectueux. Le lit peut être isolé en utilisant la ligne de court-circuit, préférablement avec un lit adjacent. Le bypass est préférentiellement effectué par le bas des adsorbeurs, permettant ainsi d'ouvrir les têtes des viroles isolées pour les décharger et les réparer. Pendant l'opération de maintenance le lit mobile simulé pourra continuer de fonctionner avec les 10 lits restants.

Avantageusement, il est possible d'ajuster la séquence de manière à maintenir la même répartition de lits d'adsorbant ou de tamis par zones. Par exemple, pour une opération normal en configuration 3 / 6 /4 / 2. La maintenance peut être opéré pendant une opération de court-circuitage en configuration 2,6 / 5,2 / 3,5 / 1,7. Avantageusement, le procédé selon la présente invention peut mettre en œuvre un déplacement synchrone des conduites d'entrée/sortie, mais peut également mettre en œuvre un déplacement non synchrone des vannes d'entrée/sortie dans le dispositif selon la présente invention, un déplacement non synchrone étant également connu sous le nom de VARICOL. Avantageusement, la production pourra être maintenue à la pureté souhaitée. Le rendement de l'opération pourra être un peu affecté par un volume plus faible de tamis à disposition.

Selon un ou plusieurs modes de réalisation, le désorbant est choisi parmi le groupe constitué par un ou plusieurs isomères de diéthylbenzène et le toluène. Selon un ou plusieurs modes de réalisation, le désorbant est le paradiéthylbenzène ou le toluène. Selon un ou plusieurs modes de réalisation, le désorbant est le toluène.

Selon un ou plusieurs modes de réalisation, l'adsorbant utilisé comprend/consiste en une Faujasite choisi parmi le groupe consistant en BaX, BaKX, et BaLSX.

Selon un ou plusieurs modes de réalisation, la charge est choisie parmi le groupe constitué par un mélange de composés essentiellement aromatiques en C8 (e.g. xylènes et éthylbenzène). Selon un ou plusieurs modes de réalisation, le mélange comprend au moins 95%, préférablement au moins 97% (e.g. au moins 99%) de composés essentiellement aromatiques en C8. Selon une ou plusieurs modes de réalisation la charge comprend au moins 15% poids de paraxylène et/ou 30% poids de métaxylène par rapport au poids total de la charge.

Un exemple de procédé de séparation LMS de grande importance industrielle concerne la séparation des coupes C8 aromatiques en vue de produire du paraxylène de pureté commerciale, typiquement à au moins 99,7% poids, et un raffinat riche en éthylbenzène, orthoxylène et métaxylène.

L'extrait produit contient du désorbant, du paraxylène et éventuellement des traces d'isomères (pureté du paraxylène supérieure à 95%, préférablement supérieur à 98%). Cet extrait peut être traité pour séparer le désorbant (e.g. par distillation) et ensuite purifié soit par cristallisation, soit par adsorption en lit mobile simulé pour augmenter la pureté du paraxylène.

Selon un ou plusieurs modes de réalisation, la température dans les lits d'adsorbant est comprise entre 140°C et 189°C et de manière préférée entre 155°C et 185°C, de manière particulièrement préférée entre 170°C et 180°C.

La pression est réglée de manière à ce que l'on reste en phase liquide en tout point du procédé selon l'invention. Selon un ou plusieurs modes de réalisation, la pression dans les lits d'adsorbant est comprise entre 1 MPa et 10 MPa, de préférence entre 2 MPa et 4 MPa, préférablement entre 2 MPa et 3 MPa.

Selon un ou plusieurs modes de réalisation, la période de permutation ST (période entre deux permutations successives des alimentations/extractions) employée est comprise entre 20 secondes et 90 secondes. Préférablement, la période de permutation ST employée est comprise entre 30 secondes et 70 secondes (e.g. 50 $\pm$ 10 secondes).

Selon un ou plusieurs modes de réalisation, le débit de circulation moyen entre les lits est compris entre 1000 m$^3$/h et 5000 m$^3$/h, préférablement compris entre 2000 m$^3$/h ou 2500 m$^3$/h et 4000 m$^3$/h, très préférablement compris entre 3000 m$^3$/h et 4000 m$^3$/h.

## Exemples

### *Dispositif de référence*

Considérons un dispositif de séparation en LMS de référence constitué de 12 adsorbeurs, chacun contenant 1 lit d'adsorbant d'un volume de 45,9 m$^3$ et d'un ratio H/D hauteur H sur diamètre D de 4.

L'écoulement est descendant dans chaque adsorbeur.

Le diamètre de chaque adsorbeur est de 3 m, la hauteur 4 entre les lignes de tangence des dômes est de 12 m.

Le débit de circulation moyen entre les lits est de 3850 m$^3$/h.

Le volume inter-lit de l'unité est estimé à 177,7 m$^3$.

### *Dispositif selon l'invention*

Considérons un dispositif de séparation en LMS selon l'invention constitué de 12 adsorbeurs, chacun contenant 1 lit d'adsorbant d'un volume de 45,9 m$^3$ et d'un ratio H/D hauteur H sur diamètre D de 4.

L'écoulement est alternativement ascendant et descendant dans les adsorbeurs successifs.

Le diamètre de chaque adsorbeur est de 3 m, la hauteur 4 entre les lignes de tangence des dômes est de 12 m.

Le débit de circulation moyen entre les lits de 3500 m$^3$/h.

Le volume inter-lit de l'unité est estimé à 125,2 m$^3$.

Cet exemple illustre notamment le gain de volume inter-lit, qui conduit à un gain sur le débit de circulation dans l'unité, donc un gain opératoire sur le coût de fonctionnement, ainsi que sur le coût de construction de l'unité (masse de métal réduite).

**Revendications**

1. Dispositif de séparation en lit mobile simulé comprenant une pluralité d'adsorbeurs (1, 2) disposés en série agencés alternativement en courant ascendant et descendant, chaque adsorbeur (1, 2) étant divisé en n chambres d'adsorption comprenant chacune un lit d'adsorbant, les n lits d'adsorbant étant séparés par n plateaux pour injecter une charge et un désorbant et soutirer un extrait et un raffinat, le dispositif comprenant au moins une ligne de court-circuit (3) adaptée pour court-circuiter au moins un adsorbeur (1, 2).

2. Dispositif selon la revendication 1, dans lequel le nombre d'adsorbeurs m est compris entre 3 et 15.

3. Dispositif selon la revendication 1 ou la revendication 2, dans lequel le nombre d'adsorbeurs m est un nombre pair.

4. Dispositif selon l'une quelconque des revendications précédentes, dans lequel le nombre n de lits d'adsorbant par adsorbeur (1, 2) est compris entre 1 et 4, préférablement compris entre 1 et 3, très préférablement compris entre 1 et 2.

5. Dispositif selon l'une quelconque des revendications précédentes, dans lequel le nombre total t de lits d'adsorbant est compris entre 6 et 24, préférablement compris entre 8 et 19, très préférablement compris entre 12 et 15.

6. Dispositif selon l'une quelconque des revendications précédentes, dans lequel les lits d'adsorbants présentent un ratio H/D hauteur H sur diamètre D égal à ou supérieur à 1, préférablement égal à ou supérieur à 1,5, très préférablement égal à ou supérieur à 2.

7. Dispositif selon l'une quelconque des revendications précédentes, dans lequel les lits d'adsorbants présentent un ratio H/D hauteur H sur diamètre D compris entre 1 et 12, préférablement compris entre 1,5 et 10, très préférablement compris entre 2 et 8.

8. Dispositif selon la revendication 1, dans lequel la ligne de court-circuit (3) est adaptée pour court-circuiter deux adsorbeurs (1, 2) adjacents.

9. Dispositif selon la revendication 1 ou la revendication 8, dans lequel la ligne de court-circuit (3) connecte un adsorbeur à courant descendant (1) à un adsorbeur à courant ascendant (2).

10. Dispositif selon l'une quelconque des revendications précédentes, dans lequel les adsorbeurs (1, 2) sont agencés en disposition verticale pour une distribution sensiblement verticale du fluide circulant dans les adsorbeurs (1, 2).

11. Dispositif selon l'une quelconque des revendications 1 à 9, dans lequel les adsorbeurs (1, 2) sont agencés en disposition horizontale pour une distribution sensiblement horizontale du fluide circulant dans les adsorbeurs (1, 2), la circulation dans les adsorbeurs (1, 2) se faisant dans un plan horizontal et suivant deux directions opposées.

12. Procédé de séparation en lit mobile simulé utilisant le dispositif de séparation en lit mobile simulé selon l'une quelconque des revendications précédentes, le procédé comprenant les étapes suivantes :

    - on alimente les adsorbeurs (1, 2) avec au moins une charge et un désorbant, et on soutire au moins un extrait et au moins un raffinat desdits adsorbeurs (1, 2), les lits d'adsorbant étant interconnectés en boucle fermée, les points d'alimentation et de soutirage dans les adsorbeurs (1, 2) étant décalés au cours du temps d'une valeur correspondant à un lit d'adsorbant avec une période de permutation et déterminant une pluralité de zones de fonctionnement du dispositif de séparation en lit mobile simulé, dont les zones principales suivantes :
    - la zone I de désorption d'un produit à séparer est comprise entre l'injection du désorbant D et le soutirage de l'extrait E ;
    - la zone II de désorption des isomères du produit à séparer est comprise entre le soutirage de l'extrait E et l'injection de la charge F ;
    - la zone III d'adsorption du produit à séparer est comprise entre l'injection de la charge F et le soutirage du raffinat R ; et
    - la zone IV est comprise entre le soutirage de raffinat R et l'injection de désorbant D.

13. Procédé selon la revendication 12, dans lequel les lits d'adsorbant sont répartis dans les zones I à IV selon des configurations dites de type a / b / c / d suivantes :

    - a est le nombre de lits en zone I;
    - b est le nombre de lits en zone II ;
    - c est le nombre de lits en zone III ; et
    - d est le nombre de lits en zone IV, et

$$a = (t * 0{,}2) * (1 \pm 0{,}2) \; ;$$

    -

$$b = (t * 0{,}4) * (1 \pm 0{,}2) \; ;$$

    -

$$c = (t * 0,27) * (1 \pm 0,2) ;$$

et

$$d = (t * 0,13) * (1 \pm 0,2),$$

et

dans lequel t est un nombre entier naturel compris entre 6 et 24, préférablement entre 8 et 15.

14. Procédé selon la revendication 12, dans lequel les lits d'adsorbant sont répartis dans les zones I à IV selon des configurations dites de type a / b / c / d suivantes :

- a est le nombre de lits en zone I;
- b est le nombre de lits en zone II ;
- c est le nombre de lits en zone III ; et
- d est le nombre de lits en zone IV, et

$$a = (t * 0,17) * (1 \pm 0,2) ;$$

$$b = (t * 0,42) * (1 \pm 0,2) ;$$

$$c = (t * 0,25) * (1 \pm 0,2) ;$$

et

$$d = (t * 0,17) * (1 \pm 0,2),$$

et

dans lequel t est un nombre entier naturel compris entre 6 et 24, préférablement entre 8 et 15.

15. Procédé selon la revendication 12, dans lequel lorsqu'on court-circuite un nombre x d'adsorbeurs, on modifie la position des points d'alimentation et des points de soutirage de sorte que la proportion prédéterminée du nombre moyen de lits d'adsorbant en opération dans les zones I à IV soit conservée selon une approximation de $\pm$ 20%, préférablement de $\pm$10%, très préférablement de $\pm$ 5%.

**Patentansprüche**

1. Vorrichtung zur Trennung mit einem simulierten Fließbett, die mehrere Adsorber (1, 2) umfasst, die in Reihe und abwechselnd mit aufsteigender und absteigender Strömung angeordnet sind, wobei jeder Adsorber (1, 2) in n Adsorptionskammern aufgeteilt ist, die jeweils ein Adsorptionsmittelbett umfassen, die n Adsorptionsmittelbetten durch n Böden getrennt sind, um ein Einsatzmaterial und ein Desorptionsmittel einzuspritzen und einen Extrakt und ein Raffinat zu entnehmen, die Vorrichtung mindestens eine Umgehungsleitung (3) umfasst, die dazu ausgelegt ist, mindestens einen Adsorber (1, 2) zu umgehen.

2. Vorrichtung nach Anspruch 1, wobei die Zahl der Absorber m zwischen 3 und 15 beträgt.

3. Vorrichtung nach Anspruch 1 oder Anspruch 2, wobei die Zahl der Absorber m eine gerade Zahl ist.

4. Vorrichtung nach einem der vorhergehenden Ansprüche, wobei die Zahl n der Adsorptionsmittelbetten pro Adsorber (1, 2) zwischen 1 und 4, vorzugsweise zwischen 1 und 3, ganz bevorzugt zwischen 1 und 2, beträgt.

5. Vorrichtung nach einem der vorhergehenden Ansprüche, wobei die Gesamtzahl t der Adsorptionsmittelbetten zwischen 6 und 24, vorzugsweise zwischen 8 und 19, ganz bevorzugt zwischen 12 und 15, beträgt.

6. Vorrichtung nach einem der vorhergehenden Ansprüche, wobei die Adsorptionsmittelbetten ein Verhältnis H/D der Höhe H zum Durchmesser D gleich oder größer als 1, vorzugsweise gleich oder größer als 1,5, ganz bevorzugt gleich oder größer als 2, aufweisen.

7. Vorrichtung nach einem der vorhergehenden Ansprüche, wobei die Adsorptionsmittelbetten ein Verhältnis H/D der Höhe H zum Durchmesser D zwischen 1 und 12, vorzugsweise zwischen 1,5 und 10, ganz bevorzugt zwischen 2 und 8, aufweisen.

8. Vorrichtung nach Anspruch 1, wobei die Umgehungsleitung (3) dazu ausgelegt ist, zwei benachbarte Adsorber (1, 2) zu umgehen.

9. Vorrichtung nach Anspruch 1 oder Anspruch 8, wobei die Umgehungsleitung (3) einen Adsorber (1) mit absteigender Strömung mit einem Adsorber (2) mit aufsteigender Strömung verbindet.

10. Vorrichtung nach einem der vorhergehenden Ansprüche, wobei die Adsorber (1, 2) in einer vertikalen Anordnung für eine im Wesentlichen vertikale Verteilung des in den Adsorbern (1, 2) zirkulierenden Fluids angeordnet sind.

11. Anordnung nach einem der Ansprüche 1 bis 9, wobei die Adsorber (1, 2) in einer horizontalen Anordnung für eine im Wesentlichen horizontale Verteilung des in den Adsorbern (1, 2) zirkulierenden Fluids angeordnet sind, wobei die Zirkulation in den Adsorbern (1, 2) in einer horizontalen Ebene und in zwei entgegengesetzten Richtungen erfolgt.

12. Verfahren zur Trennung mit einem simulierten Fließbett unter Verwendung der Trennvorrichtung mit einem simulierten Fließbett nach einem der vorhergehenden Ansprüche, wobei das Verfahren die folgenden Schritte umfasst:

- das Zuführen mindestens eines Einsatzmaterials und eines Desorptionsmittels zu den Adsorbern (1, 2) und das Entnehmen mindestens eines Extrakts und mindestens eines Raffinats von den Adsorbern (1, 2), wobei die Adsorptionsmittelbetten in einem geschlossenen Kreislauf miteinander verbunden sind, die Zufuhr- und Entnahmestellen in den Adsorbern (1, 2) im Zeitverlauf um einen einem Adsorptionsmittelbett entsprechenden Wert mit einer Umschaltdauer versetzt sind und mehrere Betriebszonen der Trennvorrichtung mit einem simulierten Fließbett mit den folgenden Hauptzonen bestimmt werden:
- der Desorptionszone I eines zu trennenden Produkts zwischen der Einspritzung des Desorptionsmittels D und der Entnahme des Extrakts E;
- der Desorptionszone II der Isomere des zu trennenden Produkts zwischen der Entnahme des Extrakts E und der Einspritzung des Einsatzmaterials F;
- der Adsorptionszone III des zu trennenden Produkts zwischen der Einspritzung des Einsatzmaterials F und der Entnahme des Raffinats R und
- der Zone IV zwischen der Entnahme des Raffinats R und der Einspritzung des Desorptionsmittels D.

13. Verfahren nach Anspruch 12, wobei die Adsorptionsmittelbetten in den Zonen I bis IV gemäß den als Typ a / b / c / d bezeichneten folgenden Konfigurationen verteilt sind:

- a ist die Zahl der Betten in Zone I;
- b ist die Zahl der Betten in Zone II;
- c ist die Zahl der Betten in Zone III und
- d ist die Zahl der Betten in Zone IV und
-

$$a = (t * 0{,}2) * (1 \pm 0{,}2);$$

-

$$b = (t * 0{,}4) * (1 \pm 0{,}2);$$

-

$$c = (t * 0{,}27) * (1 \pm 0{,}2)$$

und

-

$$d = (t * 0{,}13) * (1 \pm 0{,}2)$$

und

wobei t eine natürliche Zahl zwischen 6 und 24, vorzugsweise zwischen 8 und 15, ist.

14. Verfahren nach Anspruch 12, wobei die Adsorptionsmittelbetten in den Zonen I bis IV gemäß den als Typ a / b / c / d bezeichneten folgenden Konfigurationen verteilt sind:

- a ist die Zahl der Betten in Zone I;
- b ist die Zahl der Betten in Zone II;
- c ist die Zahl der Betten in Zone III und
- d ist die Zahl der Betten in Zone IV und
-

$$a = (t * 0{,}17) * (1 \pm 0{,}2);$$

-

$$b = (t * 0{,}42) * (1 \pm 0{,}2);$$

-

$$c = (t * 0{,}25) * (1 \pm 0{,}2)$$

und

-

$$d = (t * 0{,}17) * (1 \pm 0{,}2)$$

und

wobei t eine natürliche Zahl zwischen 6 und 24, vorzugsweise zwischen 8 und 15, ist.

15. Verfahren nach Anspruch 12, wobei bei einer Umgehung einer Zahl x von Adsorbern die Position der Zufuhrstellen und Entnahmestellen so modifiziert wird, dass der vorbestimmte Anteil der mittleren Zahl von in den Zonen I bis IV betriebenen Adsorptionsmittelbetten in einer Näherung von ± 20 %, vorzugsweise ± 10 %, ganz bevorzugt ± 5 %, gehalten wird.

**Claims**

1. Device for separation by simulated moving bed comprising a plurality of adsorbers (1, 2) which are positioned in series and arranged alternately for upflow and downflow, each adsorber (1, 2) being divided into n adsorption chambers each comprising one adsorbent bed, the n adsorbent beds being separated by n plates for injecting a feedstock and a desorbent and withdrawing an extract and a raffinate, the device comprising at least one bypass line (3) adapted to bypass at least one adsorber (1, 2).

2. Device according to Claim 1, wherein the number of adsorbers m is between 3 and 15.

3. Device according to Claim 1 or Claim 2, wherein the number of adsorbers m is an even number.

4. Device according to any one of the preceding claims, wherein the number n of adsorbent beds per adsorber (1, 2) is between 1 and 4, preferably between 1 and 3, and highly preferably between 1 and 2.

5. Device according to any one of the preceding claims, wherein the total number t of adsorbent beds is between 6 and 24, preferably between 8 and 19, and highly preferably between 12 and 15.

6. Device according to any one of the preceding claims, wherein the adsorbent beds have a height H to diameter D ratio H/D equal to or greater than 1, preferably equal to or greater than 1.5, and highly preferably equal to or greater than 2.

7. Device according to any one of the preceding claims, wherein the adsorbent beds have a height H to diameter D ratio H/D between 1 and 12, preferably between 1.5 and 10, and highly preferably between 2 and 8.

8. Device according to Claim 1, wherein the bypass line (3) is adapted to bypass two adjacent adsorbers (1, 2).

9. Device according to Claim 1 or Claim 8, wherein the bypass line (3) connects a downflow adsorber (1) to an upflow adsorber (2).

10. Device according to any one of the preceding claims, wherein the adsorbers (1, 2) are arranged in a vertical position for substantially vertical distribution of the fluid flowing through the adsorbers (1, 2).

11. Device according to any one of Claims 1 to 9, wherein the adsorbers (1, 2) are arranged in a horizontal position for substantially horizontal distribution of the fluid flowing through the adsorbers (1, 2), the flow through the adsorbers (1, 2) taking place in a horizontal plane and in two opposite directions.

12. Process for separation by simulated moving bed using the device for separation by simulated moving bed according to any one of the preceding claims, the process comprising the following steps:

- the adsorbers (1, 2) are fed with at least one feedstock and a desorbent, and at least one extract and at least one raffinate are withdrawn from said adsorbers (1, 2), the adsorbent beds being interconnected in a closed loop, the feed and withdrawal points in the adsorbers (1, 2) being shifted over the course of time by an amount corresponding to one adsorbent bed with a switching time and determining a plurality of operating zones of the device for separation by simulated moving bed, including the following main zones:
- zone I for desorption of a product to be separated is between the injection of the desorbent D and the withdrawal of the extract E;
- zone II for desorption of the isomers of the product to be separated is between the withdrawal of the extract E and the injection of the feedstock F;
- zone III for adsorption of the product to be separated is between the injection of the feedstock F and the withdrawal of the raffinate R; and
- zone IV is between the withdrawal of raffinate R and the injection of desorbent D.

13. Process according to Claim 12, wherein the adsorbent beds are distributed in zones I to IV according to the following configurations referred to as a / b / c / d type configurations:

- a is the number of beds in zone I;
- b is the number of beds in zone II;
- c is the number of beds in zone III; and
- d is the number of beds in zone IV, and
-

$$a = (t * 0.2) * (1 \pm 0.2);$$

-

$$b = (t * 0.4) * (1 \pm 0.2);$$

-

$$c = (t * 0.27) * (1 \pm 0.2);$$

and

$$d = (t * 0.13) * (1 \pm 0.2),$$

and

in which t is a natural integer between 6 and 24, preferably between 8 and 15.

14. Process according to Claim 12, wherein the adsorbent beds are distributed in zones I to IV according to the following configurations referred to as a / b / c / d type configurations:

- a is the number of beds in zone I;
- b is the number of beds in zone II;
- c is the number of beds in zone III; and
- d is the number of beds in zone IV, and

$$a = (t * 0.17) * (1 \pm 0.2);$$

$$b = (t * 0.42) * (1 \pm 0.2);$$

$$c = (t * 0.25) * (1 \pm 0.2);$$

and

$$d = (t * 0.17) * (1 \pm 0.2),$$

and

in which t is a natural integer between 6 and 24, preferably between 8 and 15.

15. Process according to Claim 12, wherein when a number x of adsorbers is bypassed, the position of the feed points and of the withdrawal points is modified so that the predetermined proportion of the average number of adsorbent beds in operation in zones I to IV is maintained according to an approximation of $\pm 20\%$, preferably of $\pm 10\%$, and highly preferably of $\pm 5\%$.

Fig. 1

Fig. 2

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2985589 A
- US 3214247 A
- US 3268605 A
- US 3592612 A
- US 4614204 A
- US 4378292 A
- US 5200075 A
- US 5316821 A
- EP 2319599 A1